# EUROPEAN PATENT SPECIFICATION

(11) **EP 3 787 585 B1**
(45) Date of publication and mention of the grant of the patent: **29.11.2023**
(21) Application number: 19744787.3
(22) Date of filing: 19.06.2019
(51) Int. Cl.: A61F 15/00

(54) **PROTECTIVE BARRIER FILMS**
SCHUTZBARRIEREFOLIEN
FILMS FORMANT BARRIÈRE PROTECTRICE

(30) Priority: 20.06.2018 GB 201810093
(43) Date of publication of application: 10.03.2021
(73) Proprietor: Packexe (Holdings) Limited, Exeter, Devon EX2 8NY (GB)
(72) Inventor: ORCHARD, Andrew Harry, Exeter Devon EX2 8NY (GB)
(74) Representative: Bryers LLP
(86) International application number: PCT/GB2019/051725
(87) International publication number: WO 2019/243819

(56) References cited:
- EP-A2- 1 871 699
- WO-A1-2012/047357
- US-A- 2 704 640
- US-A1- 2013 184 778

## Description

### Field of the Invention

The present invention relates to the dispensation of protective barrier films, in particular to a dispenser for dispensing a protective barrier film, and more particularly to a dispenser suitable for dispensing a protective barrier film in an environment in which it is desirable to maintain a sterile or aseptic condition of an item, for example within a health care environment for infection control.

### Background of the Invention

A challenge that the health care industry faces is to reduce the rates of cross-contamination between patients and/or staff as a result of using reusable medical devices. To clean such medical devices to an acceptable standard using traditional techniques can take a lot of time and resource, resulting in unnecessary downtime. There is thus a requirement for a disposable protective film layer.

For stringent infection control procedures, the barrier films must remain covered to prevent airborne contamination. However, they must also be easily accessible by medical professionals for rapid application.

It is accordingly an object of the present invention to provide an effective answer to the above problem.

It has also been recognised that a protective barrier film has beneficial uses in other applications.

Patent Publication No. US 2013/184778 A1 discloses a dispenser containing a roll of a linearly arranged array of patches, the patches connected by tear lines and the patches having a backing sheet, an adhesive layer, and a paper or cloth tab at one end that is secured to the underside of the patch by the adhesive layer. Patent Publication No. US 2704640 A discloses a tape dispensing device comprising a frame for supporting a spool of adhesive plaster tape wound around a core or hub for free rotation, the frame being a housing having opposed end walls, one of the walls having a central cylindrical flange integral therewith or otherwise secured thereto that extends axially inwardly of the housing to the other wall to form an axle for the spool and to define a finger hole by which the device may be conveniently held in an operator's hand.

### Summary of the Invention

According to a first aspect there is provided a dispenser as claimed in claim 1.

The opening may provide a tearing edge for facilitating the application of pressure to the plastic film for rupturing the perforations of a selected one of the plurality of rows of perforations. The tearing edge may be provided by a surface of an escutcheon member disposed on an exterior of the casing.

The casing may comprise a base and a cover, the cover openable to allow the protective barrier film unit to be withdrawn from and inserted into the interior of the casing. The cover may then be provided with a first part of a two-part mechanical fastening arrangement, the base is provided with the second part of the two-part mechanical fastening arrangement, and the cover is detachably attachable to the base using the two-part mechanical fastening arrangement. The cover may be clear.

The base may be provided with one or more mounting apertures for use in fixing the casing to a support surface. Alternatively, or additionally, the base may be provided with one or more non-slip feet for use in situating the casing upon a support surface.

One of the first side and the second side of the plastic film may have an adhesive coating. The adhesive coating may be provided by a solvent-based adhesive, which may be a solvent-based acrylic adhesive. Alternatively, the adhesive coating may be provided by a water-based adhesive.

The plastic film may have a thickness within the range from 20 microns to 80 microns. The plastic film may have a thickness within the range from 45 microns to 50 microns.

The rows of perforations may be spaced apart by a distance within the range from 25 mm to 300 mm. The rows of perforations may be spaced apart by a distance within the range from 75 mm to 150 mm.

The plastic film may be a low-density polyethylene film.

According to a second aspect, there is provided dispensing apparatus comprising: a dispenser according to the first aspect, and at least one further said protective barrier film unit for substituting the protective barrier film unit of the dispenser. The at least one further said protective barrier film unit may comprise a further protective barrier film unit that comprises protective film that is the same as the protective film of the protective barrier film unit of the dispenser. The at least one further said protective barrier film unit may comprise a further protective barrier film unit that comprises protective film that differs from the protective film of the protective barrier film unit of the dispenser.

A further protective barrier film unit may be identical to, or not identical to, the protective barrier film unit. A substitute protective barrier film unit may be used to simply replace the plastic film or to change, for example, one or more of the thickness, colour, strength or adhesion properties of the plastic film and/or the spacing of the rows of perforations and/or the size and/or volume of the plastic film.

Use of a dispenser according to the first aspect is in accordance with a third aspect as claimed in claim 13.

Plastic film dispensed from the protective barrier film unit may be applied to a medical device or health care item, in which use a surface of the medical device or health care item is covered by the applied plastic film.

Plastic film dispensed from the protective barrier film unit may be applied to a glass bottle having a label, in which use the label is covered by the applied plastic film.

Plastic film from the dispenser may be applied to an item to protect the item against contamination and/or damage. Plastic film from the dispenser may be used, for example, to protect all or part of an article from becoming one or more of: unclean, stained, discoloured, damp, worn, scratched, torn.

The dispenser can be used to apply a protective barrier film to an item by placing a portion of plastic film projecting through the opening of the casing on a surface of an item, with which it will remain in contact, moving the casing relative to the item to unwind plastic film from the protective barrier film unit, and then rupturing the perforations of a selected row of perforations of the withdrawn plastic film.

According to a specific example, there is provided a dispenser for use in applying a protective barrier film to medical devices, the dispenser comprising a cover containing a core supported by two end caps, a cylindrical roll of plastic film wound onto the core, one side of the film having an adhesive coating afforded by a solvent-based acrylic adhesive and the plastic film being formed with a plurality of spaced transversely extending rows of perforations, and the cover having an opening to allow dispensation of the film while simultaneously protecting the film from airborne particles. The dispenser can be designed to be wall mounted or to sit on desks to suit the needs of the medical personnel using the film. The opening through which the film is dispensed is preferably horizontal. The plastic film is preferably low-density polyethylene having a thickness within the range of 45-50 microns. The rows of perforations are preferably spaced apart by a distance within the range of from 75 mm to 150 mm.

Further particular and preferred aspects of the invention are set out in the accompanying dependent claims.

### Brief Description of the Drawings

The present invention will now be more particularly described, with reference to the accompanying drawings, in which:
**Figure 1** is a perspective view of a dispenser, in an assembled condition;
**Figure 2** is a perspective view of the dispenser of **Figure 1****,** with cover removed;
**Figure 3** is a perspective view of the dispenser of **Figure 1****,** with cover, core and plastic film removed;
**Figure 4** is an orthographic view of an underside of the dispenser of **Figure 1****;**
**Figure 5** shows a protective barrier film unit;
**Figure 6** shows dispensing apparatus;
**Figures 7 to 10** show a dispenser;
**Figure 11** illustrates a first use of the dispenser; and
**Figure 12** illustrates a second use of the dispenser.

### Description

Illustrative embodiments and examples are described below in sufficient detail to enable those of ordinary skill in the art to embody and implement the apparatus, systems and/or processes described herein. It is to be understood that embodiments and examples can be provided in many alternate forms and the invention should not be construed as limited to the embodiments and examples set forth herein but by the scope of the appended claims.

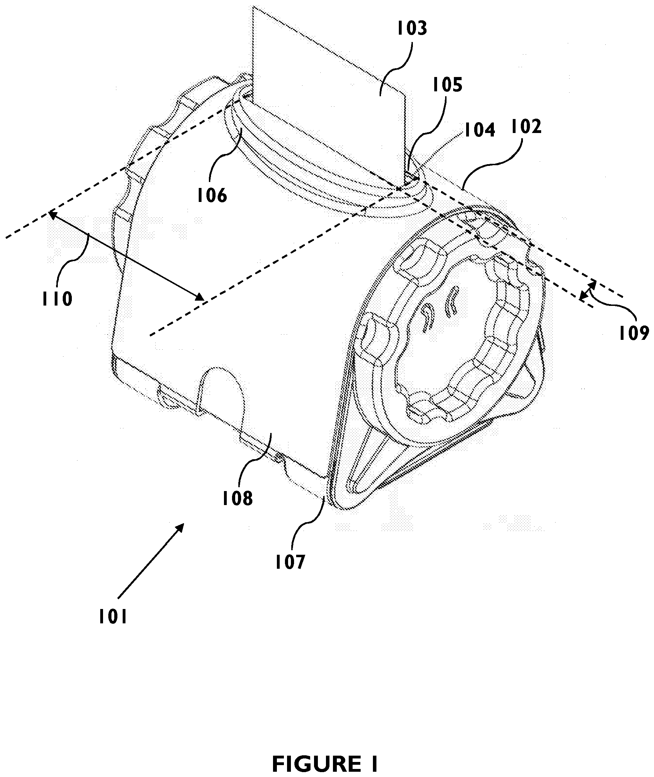

A dispenser will now be described with reference to **Figures 1 to 6****.**

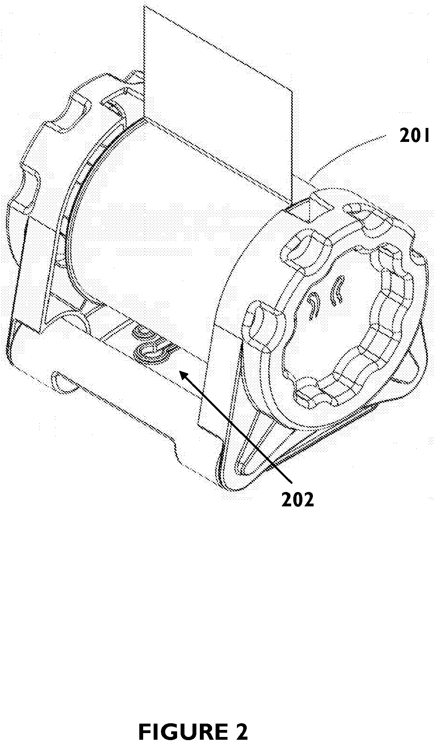

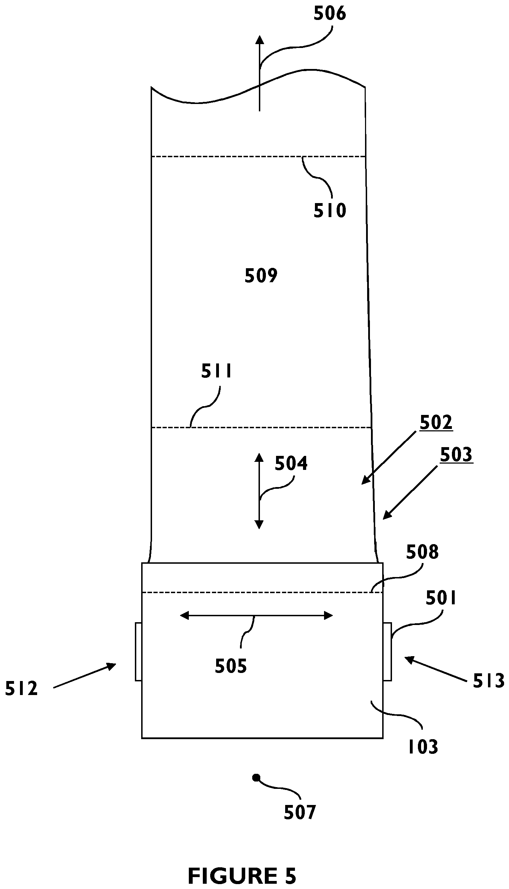

Dispenser 101 comprises a casing 102 and a protective barrier film unit 201 (see, in particular, **Figures 2** **and** **5**).

The protective barrier film unit 201 comprises a core 501 onto which a roll of a plastic film 103 is wound. The plastic film 103 is a protective barrier film. In this example, the core 501 is elongate and cylindrical. The plastic film 103 has a first side 502 and a second side 503, the second side 503 opposed to the first side 502. The roll of plastic film 103 has a length direction 504 and a transverse direction 505 perpendicular to the length direction 504. The unwind direction 506 of the roll of plastic film 103 is parallel to the length direction 504 of the roll of plastic film 103. The plastic film 103 has a thickness direction 507, which is perpendicular to the length direction 504 of the roll of plastic film 103 and to the transverse direction 506 of the roll of plastic film 103. The plastic film 103 is formed with a plurality of rows of perforations, such as rows of perforations 508 extending in the transverse direction 505 of the roll of plastic film 103 and spaced apart in the length direction 504 of the roll of plastic film 103. Sheets of the plastic film 103 are defined between successive rows of perforations, such as sheet 509 defined between the row of perforations 510 and the row of perforations 511.

As plastic film 103 is dispensed from the dispenser 101, it can be placed on a surface that requires protection and will remain in contact with that surface. Dispensed plastic film may be applied to a variety of different items, to cover all or part of that item. It is also to be understood that dispensed plastic film may be applied onto surfaces of different materials, for example, a metal, a plastic, or a fibrous material.

In an embodiment, the plastic film 103 is clear. This enables the surface on which the plastic film 103 is placed to be viewed through the plastic film 103. However, it is to be appreciated that a plastic film that is translucent or opaque, rather than transparent, may be used. In addition, a plastic film that is coloured or colour tinted, rather than uncoloured, may be used.

The plastic film 103 may be any suitable plastic film. The plastic film may be a polyethylene film.

One side of the plastic film 103 may have an adhesive coating. The adhesive coating may be provided by any suitable type of adhesive, for example a solvent-based adhesive, which may be a solvent-based acrylic adhesive, or a water-based adhesive.

The plastic film 103 may have any suitable thickness. The plastic film may have a thickness within the range from 20 microns to 80 microns.

The rows of perforations 510 may be spaced apart by any suitable distance. The rows of perforations 510 may be spaced apart by a distance within the range from 25 mm to 300 mm.

The plastic film 103 may have any suitable width and may have any suitable length.

The core 501 may have any suitable length and diameter.

The casing 102 defines an interior, indicated generally at 202, to house the protective barrier film unit 201. The casing 102 comprises a support arrangement, indicated generally at 301, to rotatably support the core 501 of the protective barrier film unit 201. The casing 102 also defines an opening 104 (see, in particular, **Figure 1**), to allow dispensing of plastic film 103 from within the interior 202 of the casing 102 while simultaneously protecting the plastic film 103 from airborne particles.

According to the present example, the opening 104 provides a tearing edge 105 for facilitating the application of pressure to the plastic film 103 for rupturing the perforations of a selected one of the plurality of rows of perforations. This enables a sheet of the plastic film 103 to be torn more easily from the protective barrier film unit 201.

In the shown example, the opening 104 extends parallel to the supported core 501.

In this specific illustrated example, the tearing edge 105 is provided by a surface of an escutcheon member 106 disposed on an exterior of the casing 102.

In the shown example, the tearing edge 105 extends parallel to the supported core 501.

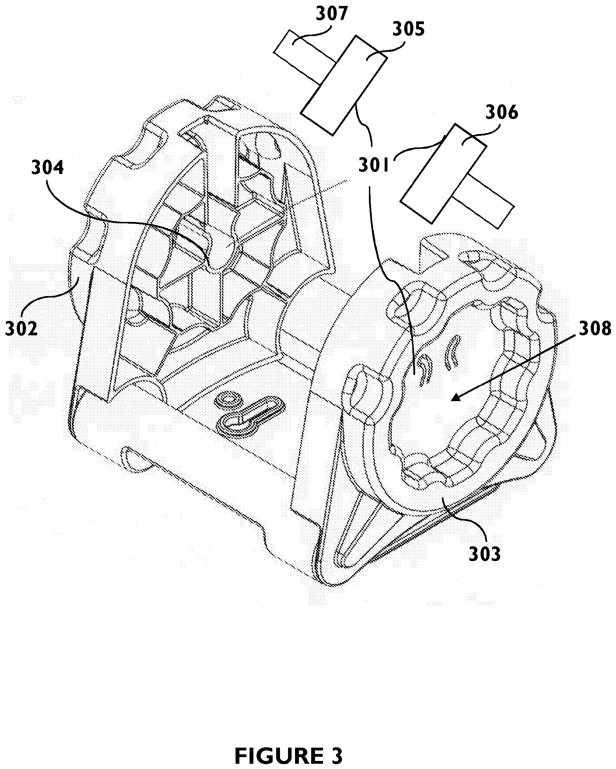

The support arrangement 301 of this specific illustrated example will now be described (see, in particular, **Figures 3** **and** **5**). The core 501 comprises first and second ends 512, 513 and the support arrangement 301 comprises first and second end caps 302, 303, each comprising a cradle, such as cradle 304 of end cap 302, and first and second capstans, 305, 306, each comprising a spindle, such as spindle 307 of capstan 305. The capstans are insertable into the ends of the protective barrier film unit and the spindles thereof are insertable into the cradles of the end caps. For example, capstan 305 is insertable into end 512 of the core 501 of the protective barrier film unit 201 and the spindle 307 thereof is insertable into the cradle 304 of end cap 302, with the other capstan 306 being insertable into the other end 513 of the core 501 of the protective barrier film unit 201 with the spindle thereof being insertable into the cradle of the other end cap 303. With the capstans 305, 306 inserted into the core 501 of the protective barrier film unit 201 and the capstans 305, 306 engaged with the end caps 302, 303, the core 501 of the protective barrier film unit 201 is received within, and rotatable within, the interior 202 of the casing 102.

In this embodiment, the support arrangement 301 and the protective barrier film unit 201 are releasably engageable, to enable a protective barrier film unit 201 to be replaced with another like protective barrier film unit 201. This enables the dispenser 101 to be refilled when the plastic film 103 of the protective barrier film unit 201 has been used up.

It is to be appreciated that any suitable alternative support arrangement for engaging the core of the protective barrier film unit in a way that allows the core of the protective barrier film unit to rotate within the casing may be utilised.

The core 501 may be made from any suitable material, for example a plastics material.

According to the present example, casing 102 comprises a base 107 and a cover 108, the cover 108 openable to allow the protective barrier film unit 201 to be withdrawn from and inserted into the interior 202 of the casing 102 (see, in particular, **Figures 1****,** **3** **and** **4**).

In the present example, the cover 108 is provided with a first part of a two-part mechanical fastening arrangement, indicated generally at 401, the base 107 is provided with the second part of the two-part mechanical fastening arrangement, and the cover 108 is detachably attachable to the base 107 using the two-part mechanical fastening arrangement.

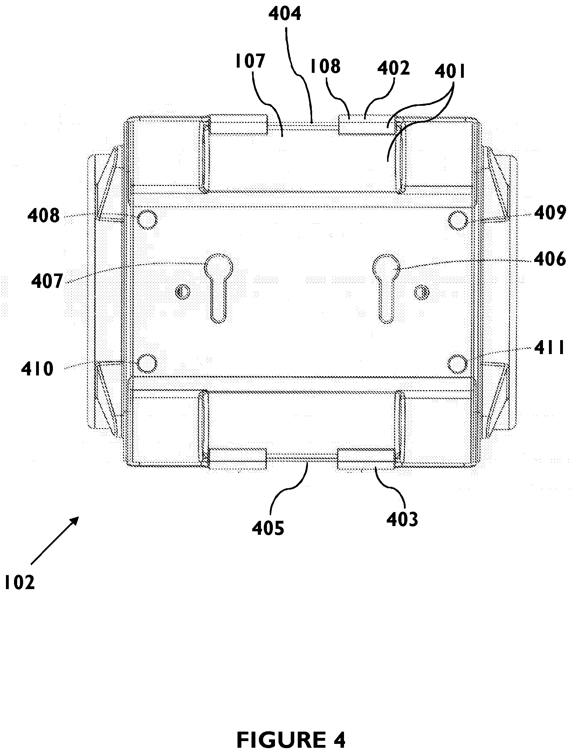

In this specific illustrated example (see, in particular **Figure 4**), the cover 108 is provided with a plurality of hooks, such as hooks 402, 403, and the base 107 is provided with a plurality of lips 404, 405 and the hooks 402, 403 latchable over respective lips 404, 405 to detachably attach the cover 108 to the base 107.

It is to be appreciated that any suitable alternative two-part mechanical fastening arrangement for detachably attaching the cover to the base may be utilised.

In an alternative example, the cover is hinged to the base. In such an example, the cover is therefore movable with respect to the base but, depending on the type of hinge, may or may not be removable therefrom.

The casing 102 may be made from any suitable material, for example a plastics material.

In an example, the cover 108 is clear. This enables the amount of plastic film 103 remaining on the core 501 to be visually determined by viewing the protective barrier film unit 201 through the cover 108.

The base 107 may be provided with one or more means to facilitate position of the casing 102 on or to one or more support surfaces. The support surface may be a substantially vertically oriented support surface, such as a wall, or may be a substantially horizontally oriented support surface, such as a desk.

According to the present example, the base 107 is provided with a plurality of mounting apertures, such as mounting apertures 406, 407, for use in securing the casing 102 at a desired position on a support surface, such as a wall (not shown).

According to the present example, also, the base 107 is provided with a plurality of non-slip feet, such as non-slip feet, such as non-slip feet 408, 409, 410 ,411, for use in situating the casing 102 upon a support surface, such as a desk (not shown).

Referring now to **Figure 1****,** and to the opening 104 defined in the casing 102, it is to be appreciated that the opening 104 is dimensioned to be large enough to avoid interfering with the free running of plastic film 103 being dispensed from the interior 202 of the casing 102 of the dispenser 101 but to be small enough to minimise the ingress of airborne contaminants into the interior 202 of the casing 102 of the dispenser 101. Thus, the opening 104 may have a width 109 that is only slightly greater than the thickness of the plastic film 103 and may have a length 110 that is only slightly greater than the width of the plastic film 103.

Referring now to **Figure 3****,** and to the end caps 302, 303, it is to be understood that the end caps 302, 303 may be designed not only to support the protective barrier film unit 201 but also to facilitate manual handling of the dispenser 101 during dispensing of plastic film 103 from the protective barrier film unit 201. In this example, the end caps 302, 303 define external recesses, such as external recess 308 of end cap 303, for receiving a hand of a user gripping the dispenser 101.

The plastic film may be a polyethylene film, which may be clear, which may have an average thickness of the order of 55 microns, a longitudinal tensile strength in excess of 1 daN/cm and a transverse tensile strength in excess of 0.7 daN/cm, and which may have an adhesion to steel in the order of 330 cN/m. The rows of perforations may be uniformly spaced apart, and may be spaced apart by a distance of the order of 100 mm, but may alternatively be spaced by a distance in the range 50mm to 150mm inclusive.

The dispenser 101 may be used in many different applications within different industries.

According to a use of the dispenser 101, plastic film from the protective barrier film unit 201 is applied to a medical device or health care item.

According to another use of the dispenser 101, plastic film from the protective barrier film unit 201 is applied to a glass bottle to cover a label thereon, for example to cover a wine bottle label.

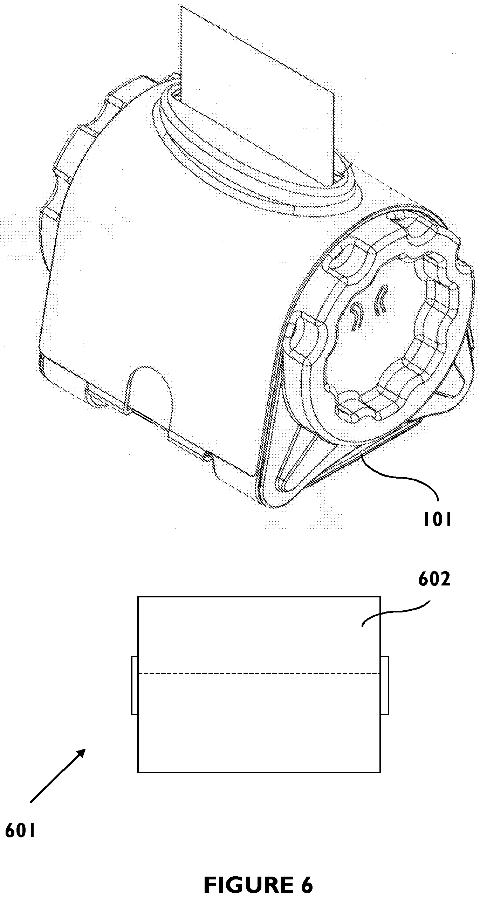

Dispensing apparatus 601 is shown in **Figure 6****,** comprising dispenser 101 and a further protective barrier film unit 602 for substituting the protective barrier film unit 201 of the dispenser 101. In an example, the further protective barrier film unit 602 is identical to the protective barrier film unit 201, and can be used to refill the dispenser 101 with the same plastic film 103 as that included in the protective barrier film unit 201. In an alternative example, the further protective barrier film unit 602 is not identical to the protective barrier film unit 201, and can be used to refill the dispenser 101 with a plastic film that differs from that included in the protective barrier film unit 201. For example, substitute protective barrier film units may be used to change, for example, one or more of the thickness, colour, strength or adhesion properties of the plastic film and/or the spacing of the rows of perforations and/or the size and/or volume of the plastic film being loaded into the dispenser. It is thus to be appreciated that a further protective barrier film unit for substituting the protective barrier film unit of the dispenser may comprise protective film that is the same as, or that differs from, the protective film of the protective barrier film unit. The core of a further protective barrier film unit may be the same as, or differ from, the core of the protective barrier film unit.

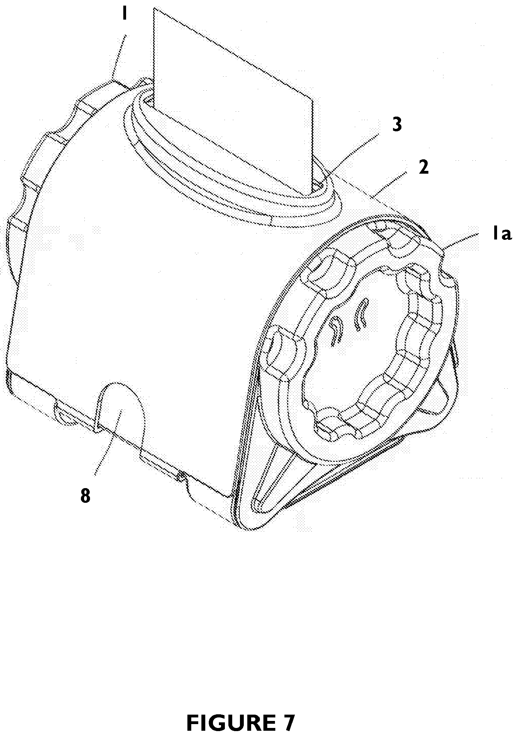

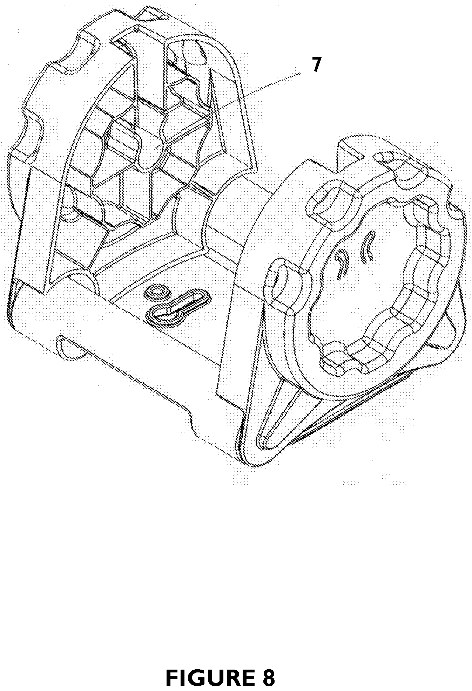

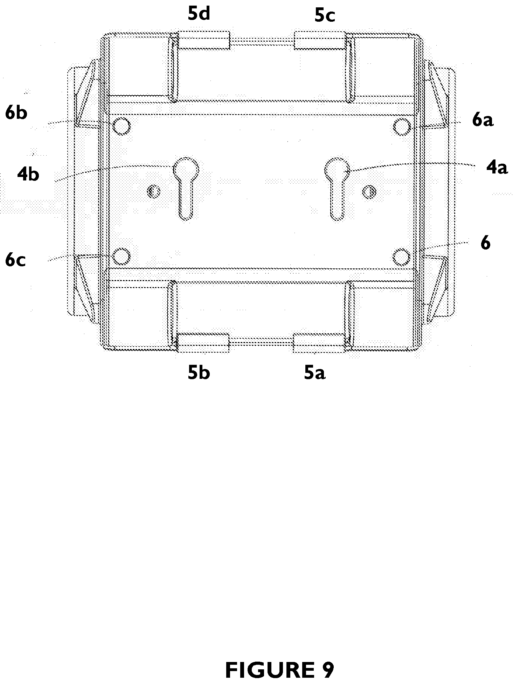

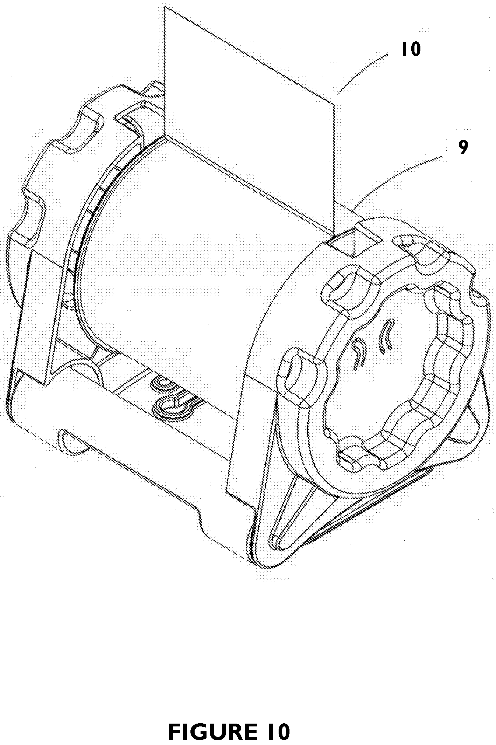

A preferred embodiment will now be described with reference to **Figures 7 to 10****,** which show the drawings of **Figures 1****,** **3****,** **4** **and** **2** respectively. The dispenser shown in **Figures 7****,** **8****,** **9** **and** **10** comprises a 100 mm wide core (9) with an internal core diameter of 75 mm, supported by two end caps (1 and 1a). A cylindrical roll of low density polyethylene film (10) is wound onto the core (9). One side of the film has an adhesive coating afforded by a solvent acrylic-based sensitive adhesive and the plastic film is formed with a plurality of spaced transversely extending rows of perforations. The plastic film is preferably low-density polyethylene having a thickness of the order of 50 microns and the rows of perforations are preferably spaced apart by a distance within the range of from 75 mm to 150 mm.

The polyethylene film (10) is a clear film which is coated on one side with a solvent-based acrylic adhesive so that, as the film is withdrawn from the roll, it can be placed on a surface that requires protection and will remain in contact with that surface.

The core (9) is mounted on two capstans, which are inserted into each end of the core and then capstan spindles are inserted into a 15 mm diameter cradle (7) in the end caps (1 and 1a) to enable rotation of the core (9).

The dispenser has a clear cover (2), with four hooks (5, 5a, 5b and 5c) which attach the cover (2) to the base of the dispenser to allow easy removal of the cover and straightforward access for refills of the rolls. The base of the cover container two semicircular arch openings (8) which provide enough purchase to remove the cover (2) from the base when using the openings to leverage the cover by hand to release the hooks. The cover (2) also has a horizontal opening (3) of 105 mm width and 12 mm height to allow the film to be pulled through when required, keeping the remainder of the roll shielded from airborne particles.

The opening (3) also provides an edge to enable the user to apply pressure to the perforations for optimal stress to facilitate rupturing of the film along a selected row of perforations and release the sheet of film from the rest of the roll.

The dispenser comes with two parallel 10 mm diameter holes (4a and 4b), which each adjoin a vertical opening of 18 mm height by 4 mm width to allow a screw head to be inserted through the hole and slid into place along the vertical opening to enable wall mounting. The base unit also contains four rubber feet (6, 6a, 6b and 6c) of 7 mm diameter to create a non-slip grip for desk mounting.

The technical data for the film may be as follows:

| Film Type | Low Density Polyethylene |
|---|---|
| Average Thickness | 55 Microns |
| Colour | Clear with colour coded logo to match surface of intended use |
| Adhesive Type | Acrylic Solvent Based |

It is to be appreciated that the casing and the protective barrier film unit may have any suitable dimensions and aesthetic appearance. The core and components of the casing may be constructed from a unitary element or a plurality of more constituent elements, each of which may be fabricated from any suitable material or combination of materials and manufactured using any suitable process or combination of processes.

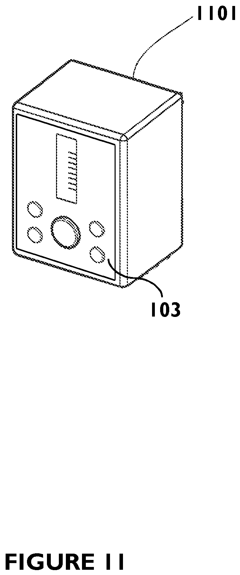

**Figure 11** illustrates use of a dispenser as described herein in a first example application, in which plastic film 103 dispensed from the protective barrier film unit is applied to a medical device or health care item 1101, wherein a surface of the medical device or health care item is covered by the applied plastic film.

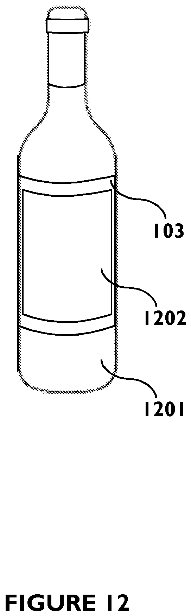

**Figure 12** illustrates use of a dispenser as described herein in a second example application, in which plastic film 103 dispensed from the protective barrier film unit is applied to a glass bottle 1201 having a label 1202, wherein the label is covered by the applied plastic film.

It is to be appreciated that the dispenser disclosed herein may be used in other applications.

Plastic film from the dispenser may be used to protect all or part of an article from becoming, for example, one or more of: unclean, stained, discoloured, damp, worn, scratched, torn.

The dispenser can be used to apply a protective barrier film to an item by placing a portion of plastic film projecting through the opening of the casing on a surface of an item, with which it will remain in contact, moving the casing relative to the item to unwind plastic film from the protective barrier film unit, and then rupturing the perforations of a selected row of perforations of the withdrawn plastic film.

Although illustrative embodiments and examples of the invention have been disclosed in detail herein, with reference to the accompanying drawings, it is to be understood that the invention is not limited to the precise embodiment and examples shown and/or described and that various changes and modifications can be effected therein by one skilled in the art without departing from the scope of the invention as defined by the appended claims.

## Claims

1. A dispenser (101) for use in applying a protective barrier film to an item, the dispenser (101) comprising:
a casing (102), and
a protective barrier film unit (201) comprising a core (501) onto which a roll of a plastic film (103) is wound, the roll of a plastic film (103) having a length direction (504) and a transverse direction (505) perpendicular to the length direction (504), the plastic film (103) having first and second sides (502, 503), and the plastic film (103) being formed with a plurality of rows of perforations (508, 510, 511) extending in the transverse direction (505) and spaced apart in the length direction (504);
the casing (102) defining an interior (202) to house the protective barrier film unit (201),
the casing (102) comprising a support arrangement (301) to rotatably support the core (501) of the protective barrier film unit (201), and
the casing (102) defining an opening (104) to allow dispensing of plastic film (103) from within the interior (202);
the opening (104) to allow dispensing of plastic film (103) from within the interior (202) while simultaneously protecting the plastic film from airborne particles;
**characterized in that**,
the core (501) comprises first and second ends (512, 513), the support arrangement (301) comprises first and second end caps (302, 303), each comprising a cradle (304), and first and second capstans (305, 306), each comprising a spindle (307);
one (305) of the first and second capstans (305, 306) is insertable into one (512) of the first and second ends (512, 513) of the core (501) of the protective barrier film unit (201) and the spindle (307) thereof is insertable into the cradle (304) of one (302) of the first and second end caps (302, 303), and
the other (306) of the first and second capstans (305, 306) is insertable into the other (513) of the first and second ends (512, 513) of the core (501) of the protective barrier film unit (201) and the spindle thereof is insertable into the cradle of the other (303) of the first and second end caps (302, 303).

2. A dispenser (101) as claimed in claim 1, wherein the opening (104) provides a tearing edge (105) for facilitating the application of pressure to the plastic film (103) for rupturing the perforations of a selected one of the plurality of rows of perforations (508, 510, 511).

3. A dispenser (101) as claimed in claim 2, wherein the tearing edge (105) is provided by a surface of an escutcheon member (106) disposed on an exterior of the casing (102).

4. A dispenser (101) as claimed in any preceding claim, wherein the casing (102) comprises a base (107) and a cover (108), the cover (108) openable to allow the protective barrier film unit (201) to be withdrawn from and inserted into the interior (202) of the casing (102).

5. A dispenser (101) as claimed in claim 4, wherein the cover (108) is provided with a first part (402) of a two-part mechanical fastening arrangement (402, 404), the base (107) is provided with the second part (404) of the two-part mechanical fastening arrangement (402, 404), and the cover (108) is detachably attachable to the base (107) using the two-part mechanical fastening arrangement (402, 404).

6. A dispenser (101) as claimed in claim 4 or claim 5, wherein the cover (108) is clear.

7. A dispenser (101) as claimed in any preceding claim, wherein the base (107) is provided with at least one of: one or more mounting apertures (406, 407) for use in fixing the casing (102) to a support surface, and one or more non-slip feet (408, 409, 410, 411) for use in situating the casing (102) upon a support surface.

8. A dispenser (101) as claimed in any preceding claim, wherein one of the first (502) side and the second side (503) of the plastic film (103) has an adhesive coating.

9. A dispenser (101) as claimed in claim 8, wherein the adhesive coating is provided by one of: a solvent-based adhesive, a solvent-based acrylic adhesive, a water-based adhesive.

10. A dispenser (101) as claimed in any preceding claim, comprising at least one of: wherein the plastic film (103) has a thickness within the range from 20 microns to 80 microns, wherein the rows of perforations are spaced apart by a distance within the range from 25 mm to 300 mm.

11. A dispenser (101) as claimed in any preceding claim, wherein the end caps (302, 303) define external recesses (308) for receiving a hand of a user gripping the dispenser (101).

12. Dispensing apparatus comprising:
a dispenser (101) as claimed in any of claims 1 to 11, and
at least one further said protective barrier film unit (602) for substituting the protective barrier film unit (201) of the dispenser (101).

13. Use of a dispenser (101) as claimed in any of claims 1 to 11.

14. Use of a dispenser (101) as claimed in claim 13, in which plastic film (103) dispensed from the protective barrier film unit (201) is applied to a medical device or health care item (1101), wherein a surface of the medical device or health care item (1101) is covered by the applied plastic film (103).

15. Use of a dispenser (101) as claimed in claim 14, in which plastic film (103) dispensed from the protective barrier film unit (201) is applied to a glass bottle (1201) having a label (1202), wherein the label (1202) is covered by the applied plastic film (103).

## Patentansprüche

1. Ein Spender (101) zur Verwendung beim Aufbringen einer Schutzfolie auf einen Gegenstand, wobei der Spender (101) aufweist:
ein Gehäuse (102), und
eine Schutzfolieneinheit (201), mit einem Kern (501), auf den eine Rolle Kunststofffolie (103) gewickelt ist, wobei die Rolle Kunststofffolie (103) eine Längsrichtung (504) und eine zur Längsrichtung (504) senkrechte Querrichtung (505) aufweist, die Kunststofffolie (103) eine erste und eine zweite Seite (502, 503) aufweist, und die Kunststofffolie (103) mit einer Mehrzahl an sich in der Querrichtung (505) erstreckenden und in der Längsrichtung (504) beabstandeten Reihen von Perforationen (508, 510, 511) ausgebildet ist; wobei
das Gehäuse (102) zur Aufnahme der Schutzfolieneinheit (201) einen Innenraum (202) definiert;
das Gehäuse (102) eine Stützanordnung (301) aufweist, um den Kern (501) der Schutzfolieneinheit (201) drehbar zu stützen, und
das Gehäuse (102) eine Öffnung (104) definiert, um das Ausgeben von Kunststofffolie (103) aus dem Innenraum (202) zu ermöglichen;
wobei die Öffnung (104) die Ausgabe von Kunststofffolie (103) aus dem Innenraum (202) ermöglicht und gleichzeitig die Kunststofffolie vor luftgetragenen Partikeln schützt;
**dadurch gekennzeichnet, dass**,
der Kern (501) ein erstes und ein zweites Ende (512, 513) aufweist, die Stützanordnung (301) eine erste und eine zweite Endkappe (302, 303) aufweist, mit jeweils einer Aufnahme (304) und jeweiligem ersten und zweiten jeweils eine Spindel (307) aufweisenden Kapstan (305, 306); wobei einer (305) der ersten oder der zweiten Kapstane (305, 306) in eines (512) der ersten und zweiten Enden (512, 513) des Kerns (501) der Schutzfolieneinheit 25 (201) einführbar ist, und dessen Spindel (307) in die Aufnahme (304) einer (302) der ersten und zweiten Endkappen (302, 303) einführbar ist, und der andere (306) der ersten und zweiten Kapstane (305, 306) in das andere (513) der ersten und zweiten Enden (512, 513) des Kerns (501) der Schutzfolieneinheit (201) einführbar ist, und dessen Spindel in die Aufnahme der anderen (303) der ersten und zweiten Endkappen (302, 303) einführbar ist.

2. Ein Spender (101) nach Anspruch 1, wobei die Öffnung (104) eine Abreißkante (105) aufweist, um die Ausübung von Druck auf die Kunststofffolie (103) zu erleichtern, um die Perforationen einer ausgewählten Perforationsreihe aus der Mehrzahl an Perforationsreihen (508, 510, 511) aufzureißen.

3. Ein Spender (101) nach Anspruch 2, wobei die Abreißkante (105) von einer Oberfläche eines an der Außenseite des Gehäuses (102) angeordneten Beschlagteils (106) gebildet wird.

4. Ein Spender (101) nach einem der vorherigen Ansprüche, wobei das Gehäuse (102) eine Basis (107) und eine Abdeckung (108) umfasst, wobei die Abdeckung (108) geöffnet werden kann, um zu ermöglichen, dass die Schutzfolieneinheit (201) aus dem Innenraum (202) des Gehäuses (102) herausgenommen und in diesen eingeführt werden kann.

5. Ein Spender (101) nach Anspruch 4, wobei die Abdeckung (108) mit einem ersten Teil (402) einer zweiteiligen mechanischen Befestigungsanordnung (402, 404) versehen ist, die Basis (107) mit dem zweiten Teil (404) der zweiteiligen mechanischen Befestigungsanordnung (402, 404) versehen ist, und die Abdeckung (108) unter Verwendung der zweiteiligen mechanischen Befestigungsanordnung (402, 404) an der Basis (107) abnehmbar befestigt werden kann.

6. Ein Spender (101) nach Anspruch 4 oder Anspruch 5, wobei die Abdeckung (108) durchsichtig ist.

7. Ein Spender (101) nach einem der vorherigen Ansprüche, wobei die Basis (107) mit zumindest einem der folgenden Elemente versehen ist: einer oder mehreren Montageöffnungen (406, 407) zur Verwendung bei der Befestigung des Gehäuses (102) an einer Auflagefläche, und einem oder mehreren rutschfesten Füßen (408, 409, 410, 411) zur Verwendung bei der Aufstellung des Gehäuses (102) auf einer Auflagefläche.

8. Ein Spender (101) nach einem der vorherigen Ansprüche, wobei eine der ersten (502) und der zweiten Seite (503) der Kunststofffolie (103) eine Klebstoffbeschichtung aufweist.

9. Ein Spender (101) nach Anspruch 8, wobei die Klebstoffbeschichtung durch einen der folgenden Klebstoffe bereitgestellt wird: einen lösungsmittelbasierten Klebstoff, einen lösungsmittelbasierten Acrylklebstoff, einen wasserbasierten Klebstoff.

10. Ein Spender (101) nach einem der vorherigen Ansprüche, aufweisend zumindest eines der folgenden Merkmale: wobei die Kunststofffolie (103) eine Dicke im Bereich von 20 µm bis 80 µm aufweist, wobei die Perforationsreihen in einem Abstand im Bereich von 25 mm bis 300 mm voneinander angeordnet sind.

11. Ein Spender (101) nach einem der vorherigen Ansprüche, wobei die Endkappen (302, 303), zum Angreifen einer Hand eines den Spender (101) bedienenden Benutzers, äußere Aussparungen (308) aufweisen.

12. Spendevorrichtung, aufweisend:
einen Spender (101) nach einem der Ansprüche 1 bis 11, und
zumindest eine weitere Schutzfolieneinheit (602) zum Ersetzen der Schutzfolieneinheit (201) des Spenders (101).

13. Verwendung eines Spenders (101) nach einem der Ansprüche 1 bis 11.

14. Verwendung eines Spenders (101) nach Anspruch 13, bei dem eine von der Schutzfolieneinheit (201) ausgegebene Kunststofffolie (103) auf eine medizinische Vorrichtung oder einen Gegenstand der Gesundheitsversorgung (1101) aufgebracht wird, wobei eine Oberfläche der medizinischen Vorrichtung oder des Gegenstands der Gesundheitsversorgung (1101) von der aufgebrachten Kunststofffolie (103) überdeckt wird.

15. Verwendung eines Spenders (101) nach Anspruch 14, bei dem eine von der Schutzfolieneinheit (201) ausgegebene Kunststofffolie (103) auf eine mit einem Etikett (1202) versehene Glasflasche (1201) aufgebracht wird, wobei das Etikett (1202) von der aufgebrachten Kunststofffolie (103) überdeckt wird.

## Revendications

1. Distributeur (101) destiné à être utilisé pour appliquer un film barrière protecteur sur un article, le distributeur (101) comprenant :
un boîtier (102) et
une unité formant film barrière de protection (201) comprenant un noyau (501) sur lequel un rouleau d'un film plastique (103) est enroulé, le rouleau d'un film plastique (103) ayant une direction longitudinale ( 504) et une direction transversale (505) perpendiculaire à la direction longitudinale (504), le film plastique (103) ayant des premier et deuxième côtés (502, 503), et le film plastique (103) étant formé avec une pluralité de rangées de perforations (508, 510, 511) s'étendant dans la direction transversale (505) et espacées l'une de l'autre dans la direction longitudinale (504) ;
le boîtier (102) définissant un intérieur (202) pour loger l'unité formant film barrière de protection (20) le boîtier (102) comprenant un agencement de support (301) pour supporter de manière rotative le noyau (501) de l'unité formant film barrière de protection (20 1) et le boîtier (102) définissant une ouverture (104) pour permettre la distribution de film plastique (103) depuis l'intérieur (202) ;
l'ouverture (104) permettant la distribution du film plastique (103) depuis l'intérieur (202) tout en protégeant simultanément le film plastique des particules en suspension dans l'air ;
**caractérisé en ce que**
le noyau (501) comprend des première et deuxième extrémités (512, 513), l'agencement de support (301) comprend des premier et deuxième capuchons d'extrémité (302, 303), comprenant chacun un berceau (304), et des premier et deuxième cabestans (305, 306), comprenant chacun une broche (307) ;
l'un (305) des premier et deuxième cabestans (305, 306) peut être inséré dans l'une (512) des première et deuxième extrémités (512, 513) du noyau (501) de l'unité formant film barrière de protection (201) et sa broche (307) peut être insérée dans le berceau (304) de l'un (302) des premier et deuxième capuchons d'extrémité (302, 303), et de l'autre (306) des premier et deuxième cabestans (305, 306) peut être inséré dans l'autre (513) des première et deuxième extrémités (512, 513) du noyau (501) de l'unité formant film barrière de protection (201) et sa broche peut être insérée dans le berceau de l'autre (303) des premier et deuxième capuchons d'extrémité (302, 303).

2. Distributeur (101) selon la revendication 1, dans lequel l'ouverture (104) fournit un bord de déchirure (105) pour faciliter l'application d'une pression sur le film plastique (103) pour rompre les perforations d'une rangée sélectionnée parmi la pluralité de rangées de perforations (508, 510, 511).

3. Distributeur (101) selon la revendication 2, dans lequel le bord de déchirure (105) est fourni par une surface d'un élément d'écusson (106) disposé sur un extérieur du boîtier (102).

4. Distributeur (101) selon l'une quelconque des revendications précédentes, dans lequel le boîtier (102) comprend une base (107) et un couvercle (108), le couvercle (108) pouvant être ouvert pour permettre à l'unité formant film barrière de protection (201) d'être retiré et inséré à l'intérieur (202) du boîtier (102).

5. Distributeur (101) selon la revendication 4, dans lequel le couvercle (108) est pourvu d'une première partie (402) d'un agencement de fixation mécanique en deux parties (402, 404), la base (107) est pourvue de la deuxième partie (404) de l'agencement de fixation mécanique en deux parties (402, 404), et le couvercle (108) peut être fixé de manière amovible à la base (107) à l'aide de l'agencement de fixation mécanique en deux parties (402, 404).

6. Distributeur (101) selon la revendication 4 ou la revendication 5, dans lequel le couvercle (108) est transparent.

7. Distributeur (101) selon l'une quelconque des revendications précédentes, dans lequel la base (107) est dotée d'au moins l'un des éléments suivants : une ou plusieurs ouvertures de montage (406, 407) destinées à être utilisées pour fixer le boîtier (102) à une surface de support, et un ou plusieurs pieds antidérapants (408, 409, 410, 411) destinés à être utilisés pour placer le boîtier (102) sur une surface de support.

8. Distributeur (101) selon l'une quelconque des revendications précédentes, dans lequel l'un parmi le premier (502) côté et le deuxième côté (503) du film plastique (103) a un revêtement adhésif.

9. Distributeur (101) selon la revendication 8, dans lequel le revêtement adhésif est fourni par l'un des éléments suivants : un adhésif à base de solvant, un adhésif acrylique à base de solvant, ou un adhésif à base d'eau.

10. Distributeur (101) selon l'une quelconque des revendications précédentes, comprenant au moins l'un des éléments suivants : le film plastique (103) a une épaisseur comprise dans la plage de 20 microns à 80 microns, dans lequel les rangées de perforations sont espacées d'une distance comprise entre 25 mm et 300 mm.

11. Distributeur (101) selon l'une quelconque des revendications précédentes, dans lequel les capuchons d'extrémité (302, 303) définissent des évidements externes (308) pour recevoir la main d'un utilisateur saisissant le distributeur (101).

12. Appareil distributeur comprenant :
un distributeur (101) selon l'une quelconque des revendications 1 à 11, et
au moins une autre dite unité formant film barrière de protection (602) pour remplacer l'unité formant film barrière de protection (201) du distributeur (101).

13. Utilisation d'un distributeur (101) selon l'une quelconque des revendications 1 à 11.

14. Utilisation d'un distributeur (101) selon la revendication 13, dans lequel le film plastique (103) distribué à partir de l'unité formant film barrière de protection (201) est appliqué sur un dispositif médical ou un article de soins de santé (1101), dans lequel une surface du dispositif médical ou l'article de soins de santé (1101) est recouvert par le film plastique appliqué (103).

15. Utilisation d'un distributeur (101) selon la revendication 14, dans lequel le film plastique (103) distribué à partir de l'unité formant film barrière de protection (201) est appliqué sur une bouteille en verre (1201) comportant une étiquette (1202), l'étiquette (1202) étant recouverte par le film plastique appliqué (103).
